# EUROPEAN PATENT APPLICATION

(11) **EP 1 403 268 A1**
(43) Date of publication of application: **31.03.2004**
(21) Application number: 02738686.1
(22) Date of filing: 13.06.2002
(51) Int. Cl.: C07D 405/14, C07D 413/14, C09K 11/06, G01N 21/78, G01N 31/00, G01N 31/22

(54) **FLUORESCENT PROBES FOR ZINC**

(30) Priority: 14.06.2001 JP 2001179627
(71) Applicant: DAIICHI PURE CHEMICALS CO. LTD., Chuo-ku Tokyo 103-0027 (JP); Nagano, Tetsuo, Suginami-ku, Tokyo 167-0032 (JP)
(72) Inventor: NAGANO, Tetsuo, Suginami-ku, Tokyo 167-0032 (JP); KIKUCHI, Kazuya, Sakae-ku, Yokohama-shi, Kanagawa 247-000 (JP); HIRANO, Tomoya, Nerima-ku, Tokyo 176-0022 (JP); MARUYAMA, Satoko, Kamakura-shi, Kanagawa 247-0053 (JP)
(74) Representative: Sternagel, Fleischer, Godemeyer & Partner Patentanwälte
(86) International application number: PCT/JP2002/005900
(87) International publication number: WO 2002/102795

(57) **Abstract**

A compound represented by the following general formula (I) or a salt thereof: 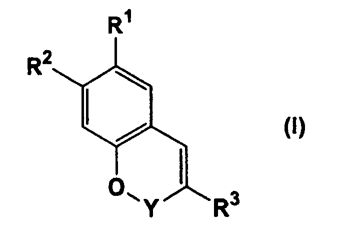 wherein R¹ represents hydrogen atom, an alkyl group, an alkoxy group, or hydroxy group; R² represents a group represented by the following formula (A): 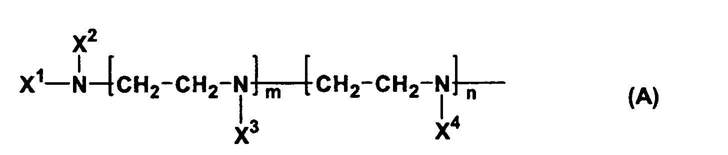 wherein X¹ to X⁴ represent hydrogen atom, an alkyl group, or 2-pyridylmethyl group, and m and n represent 0 or 1; Y represents a single bond or -CO-; R³ represents a carboxy-substituted aryl group, a carboxy-substituted heteroaryl group, benzothiazol-2-yl group, or 5-oxo-2-thioxo-4-imidazolyzinylidenmethyl group], and a fluorescent probe for zinc which comprises said compound or a salt thereof.

## Description

### Technical Field

The present invention relates to a fluorescent probe for specifically trapping a zinc ion.

### Background Art

Zinc is an essential metallic element that is present in the human body in the largest amount next to iron. Most zinc ions in cells strongly couple to proteins and are involved in the maintenance of structure or in the expression of function of the protein. Various reports have been also made on the physiological role of free zinc ions, which are present in the cell in a very small quantity (generally at a level of µM or lower). In particular, zinc ions are considered to be significantly involved in one type of cell death, i.e., apoptosis, and it is reported that zinc ions accelerate senile plaque formation in Alzheimer's disease.

A compound (a fluorescent probe for zinc), which specifically traps a zinc ion to form a complex and emits fluorescence upon the formation of the complex, has been conventionally used to measure zinc ions in tissue. For example, TSQ (Reyes, J.G., et al., Biol. Res., 27, 49, 1994), Zinquin ethyl ester (Tsuda, M. et al., Neurosci., 17, 6678, 1997), Dansylaminoethylcyclen (Koike, T. et al., J. Am. Chem. Soc., 118, 12686, 1996), and Newport Green (a catalog of Molecular Probe: "Handbook of Fluorescent Probes and Research Chemicals" 6th Edition by Richard P. Haugland pp. 531-540) have been used practically as fluorescent probes for zinc.

As a highly sensitive fluorescent probe for zinc which has overcome defects of the conventional fluorescent probes such as TSQ, the present inventors have provided a probe which has a cyclic amine or a polyamine as a substituent, and traps zinc ions to emit intensive fluorescence with long wavelength excitation light (Japanese Patent Unexamined Publication No. 2000-239272). The present inventors have also provided a probe which quickly reacts with a zinc ion to form a fluorescent complex, enabling a measurement of zinc in organisms with excellent accuracy and high sensitivity (J. Am. Chem. Soc., 2000, 122, 12399-12400).

When a fluorescent probe is applied to cells, concentrations of the fluorescent probe introduced into cells may sometimes vary depending on types of cells. There are also many factors which influence measurements, such as possibilities that thickness of cell membranes may cause differences of fluorescence intensities in area to be measured, and fluorescent probes may localize in highly hydrophobic moiety such as membranes.

As a method that can reduce measurement errors caused by these factors to achieve a precise quantitative measurement, the ratio measurement method has been developed and used (Kawanishi Y., et al., Angew. Chem. Int. Ed., 39(19), 3438, 2000). The method comprises a step of measuring fluorescence intensities at different two wavelengths in a fluorescence spectrum or an excitation spectrum to detect the ratio of the intensities. According to the method, the influences of concentrations of a fluorescent probe, per se, and the excitation light intensities are negligible, and measurement errors that are derived from localizations, changes in concentration, or discolorations of a fluorescent probe itself can also be eliminated.

For example, as a fluorescent probe for measuring a calcium ion, Fura 2 (1-[6-amino-2-(5-carboxy-2-oxazolyl)-5-benzofuranyloxy]-2-(2-amino-5-methylphenoxy) ethane-N,N,N',N'-tetraacetic acid, pentapotassium salt: Dojindo Laboratories 21st edition/ general catalog, 137-138, published on April 20, 1998, DOJINDO LABORATORIES Corporation) has been practically used. The compound has a feature that a peak of an excitation wavelength shifts to shorter wavelength by binding to a calcium ion. When the compound is excited at around 335 nm, the fluorescence intensity increases with increase of the concentration of calcium ions, whereas when the compound is excited at around 370 to 380 nm, the fluorescence intensity reduces with increase of the concentration of calcium ions. Therefore, by excitations of the compound at two suitable wavelengths, and by calculation of the ratio of the fluorescence intensities at the two wavelengths, calcium ions can be precisely measured irrespective of probe concentration, light source intensity, the size of cells, and the like.

In addition, by using the feature of the aforementioned Fura 2 or structurally similar compounds to trap ions other than a calcium ion, an application of the compounds for detecting zinc ions was studied and reported (Hyrc K.L., et al., Cell Calcium, 27(2), 75, 2000).

However, as for a fluorescent probe for zinc, a probe that can give a sufficient wavelength shift in an excitation spectrum or a fluorescence spectrum by specifically binding to a zinc ion has not been developed so far. Therefore, the ratio method has not been applicable for a precise measurement of an intracellular zinc ion.

### Disclosure of the Invention

An object of the present invention is to provide a compound or a salt thereof which can be used as a highly sensitive fluorescent probe for zinc. More specifically, the object of the present invention is to provide a compound which can specifically trap zinc ions and give a wavelength shift of a peak in an excitation spectrum or a fluorescence spectrum by trapping a zinc ion. Another object of the present invention is to provide a compound which can be used as a fluorescent probe for measuring a zinc ion by the ratio measurement method. Further, an object of the present invention is to provide a fluorescent probe for zinc which comprises a compound with the aforementioned characteristic features, and a method for measuring a zinc ion in which said fluorescent probe for zinc is used.

The inventors of the present invention conducted various studies to achieve the foregoing objects. As a result, they found that a compound represented by the following general formula (I) can specifically trap a zinc ion, and give a remarkable wavelength shift of a peak in an excitation spectrum, and by using said compound, a zinc ion can be measured with excellent accuracy by the ratio measurement method. The present invention was achieved on the basis of these findings.

The present invention thus provides a compound represented by the following general formula (I) or a salt thereof: 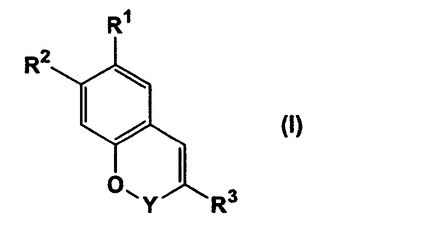 [wherein R¹ represents hydrogen atom, an alkyl group, an alkoxy group, or hydroxy group; R² represents a group represented by the following formula (A): 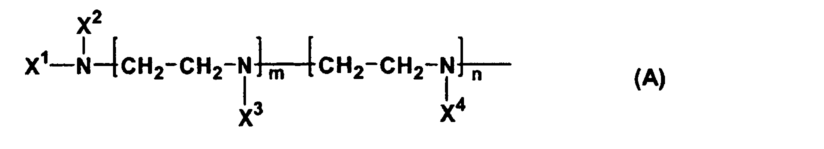 (wherein X¹, X², X³, and X⁴ each independently represents hydrogen atom, an alkyl group, or 2-pyridylmethyl group, and m and n each independently represents 0 or 1); Y represents a single bond or -CO-; R³ represents a carboxy-substituted aryl group, a carboxy-substituted heteroaryl group, benzothiazol-2-yl group, or 5-oxo-2-thioxo-4-imidazolyzinylidenmethyl group].

As a preferred embodiment of the present invention, provided are the aforementioned compound or a salt thereof wherein m is 0 or 1 and n is 0; the aforementioned compound or a salt thereof wherein both of X¹ and X² are 2-pyridylmethyl groups, and when m is 1, X³ is hydrogen atom; the aforementioned compound or a salt thereof wherein Y is a single bond; the aforementioned compound or a salt thereof wherein R¹ is methoxy group; the aforementioned compound or a salt thereof wherein R³ is a carboxy-substituted aryl group or a carboxy-substituted heteroaryl group; the aforementioned compound or a salt thereof wherein the carboxyl group substituting on the aryl ring or the heteroaryl ring has a protective group; and the aforementioned compound or a salt thereof wherein the carboxyl group having a protective group is methoxycarbonyl group or acetoxymethyloxycarbonyl group.

As particularly preferred embodiments of the present invention, provided are the aforementioned compound or a salt thereof wherein m is 1, n is 0, both of X¹ and X² are 2-pyridylmethyl groups, X³ is hydrogen atom, Y is a single bond, R¹ is methoxy group, and R³ is p-carboxyphenyl group;
the aforementioned compound or a salt thereof wherein m is 1, n is 0, both of X¹ and X² are 2-pyridylmethyl groups, X³ is hydrogen atom, Y is a single bond, R¹ is methoxy group, and R³ is 5-carboxyoxazol-2-yl group;
the aforementioned compound or a salt thereof wherein m is 0, n is 0, both of X¹ and X² are 2-pyridylmethyl groups, Y is a single bond, R¹ is methoxy group, and R³ is 5-carboxyoxazol-2-yl group;
the aforementioned compound or a salt thereof wherein m is 1, n is 0, both of X¹ and X² are 2-pyridylmethyl groups, X³ is hydrogen atom, Y is a single bond, R¹ is methoxy group, and R³ is p-acetoxymethyloxycarbonylphenyl group;
the aforementioned compound or a salt thereof wherein m is 1, n is 0, both of X¹ and X² are 2-pyridylmethyl groups, X³ is hydrogen atom, Y is a single bond, R¹ is methoxy group, and R³ is 5-acetoxymethyloxycarbonyloxazol-2-yl group; and
the aforementioned compound or a salt thereof wherein m is 0, n is 0, both of X¹ and X² are 2-pyridylmethyl groups, Y is a single bond, R¹ is methoxy group, and R³ is 5-acetoxymethyloxycarbonyloxazol-2-yl group.

From another aspect, the present invention provides a fluorescent probe for zinc which comprises the aforementioned compound or a salt thereof; a zinc complex which is formed with the aforementioned compound or a salt thereof and a zinc ion; and an agent for measuring zinc ions which comprises the aforementioned compound or a salt thereof.

From further aspect of the present invention, provided are a method for measuring zinc ions which comprises the following steps of:
(a) reacting the aforementioned compound or a salt thereof with a zinc ion; and
(b) measuring fluorescence intensity of a zinc complex produced in the above step (a); and the aforementioned method wherein the measurement is conducted by the ratio method.

Still further, provided are a method for measuring zinc ions which comprises the following steps of:
(a) allowing the aforementioned compound with the carboxyl group protected or a salt thereof to be taken up into cells; and
(b) measuring fluorescence intensity of a zinc complex produced by a reaction, with zinc ion, of a compound or a salt thereof (provided that the carboxyl group does not have a protective group)which is generated by hydrolysis of said compound or a salt thereof after being taken up into cells;
and the aforementioned method wherein the measurement is conducted by imaging.

### Brief Explanation of Drawings

Fig. 1 shows changes in excitation spectra of the compound of the present invention (Compound (11)) depending on concentration of zinc ions.
Fig. 2 shows the result of the ratiometric measurement of zinc ions and other metal ions by using Compound (11). In the figure, (A) shows a change in the ratio by addition of zinc ions, and (B) shows changes in the ratio by addition of ions other than zinc ion.
Fig. 3 shows spectral characteristics of the compound of the present invention. a) Changes in UV spectra of Compound (14), b) Excitation spectra of Compound (14) with fluorescence wavelengths fixed to 495 nm, c) Changes in UV spectra of Compound (11), d) Excitation spectra of Compound (14) with fluorescence wavelengths fixed at 530 nm.
Fig. 4 shows spectral characteristics of the compound of the present invention. a) Fluorescence spectra of Compound (15) with excitation wavelengths fixed at 325 nm, b) Excitation spectra of Compound (15) with fluorescence wavelengths fixed at 445 nm.
Fig. 5 shows changes in concentration of intracellular zinc ions when Compound (13) is used, which are shown as changes in the ratios of fluorescence intensity on excitations at 340 nm and 380 nm with fluorescent microscope. At the time point of Arrow (1), 150 µM of zinc sulfate and 15 µM of pyrithione were added, and at the time point of Arrow (2), 400 µM of TPEN was added
Fig. 6 shows image of transmitted light through cells and changes in fluorescence intensity ratio when Compound (13) was used. In the figure, (a) shows a result of transmitted light image, (b) shows a result before stimulation, (c) shows a result after increasing concentration of intracellular zinc ions by using zinc sulfate and pyrithione, (d) shows a result after lowering concentration of intracellular zinc ions by using TPEN.

### Best Mode for Carrying out the Invention

"An alkyl group" or an alkyl moiety of a substituent containing the alkyl moiety (for example, an alkoxy group) used in the specification means, for example, a linear, branched, or cyclic alkyl group, or an alkyl group comprising a combination thereof having 1 to 12 carbon atoms, preferably 1 to 6 carbon atoms, and more preferably 1 to 4 carbon atoms. More specifically, a lower alkyl group (an alkyl group having 1 to 6 carbon atoms) is preferred as the alkyl group. Examples of the lower alkyl groups include methyl group, ethyl group, n-propyl group, isopropyl group, cyclopropyl group, n-butyl group, sec-butyl group, isobutyl group, tert-butyl group, cyclopropylmethyl group, n-pentyl group, and n-hexyl group. Methyl group is preferable as the alkyl group represented by R¹, and methoxy group is preferable as the alkoxy group represented by R¹. Methoxy group is particularly preferable. As the alkyl group represented by X¹, X², X³, and X⁴ in R², methyl group is preferable.

As an aryl group in the carboxy substituted aryl group represented by R³, a monocyclic or condensed aryl group may be used. For example, phenyl group, naphthyl group, and the like are preferable. Phenyl group is particularly preferable. As a heteroaryl group in the carboxy substituted heteroaryl group represented by R³, a monocyclic or condensed heteroaryl group may be used. The number and the type of hetero atom in the heteroaryl group are not particularly limited. For example, the heteroaryl group may contain 1 to 4, preferably 1 to 3, more preferably 1 or 2 hetero atoms selected from the group consisting of nitrogen atom, oxygen atom, and sulfur atom. Examples of heteroaryl compounds that constitute the heteroaryl group include, but not limited to, oxazole, imidazole, thiazole, benzoxazole. benzimidazole, and benzthiazole. Oxazolyl group is preferable as the heteroaryl group.

In the carboxy substituted aryl group or the carboxy substituted heteroaryl group represented by R³, the number of the carboxy groups substituting on the aryl ring or the heteroaryl ring is not particularly limited, and preferably 1 to 2, most preferably 1. The carboxy group substituting on the aryl ring or the heteroaryl ring may have a protective group. As for the protective groups for carboxyl groups, "Protective Groups in Organic Synthesis," (T. W. Greene, John Wiley & Sons, Inc. (1981)) and the like can be referred to. Preferable examples of the carboxyl group having a protective group include esters, particularly alkoxycarbonyl groups. A particularly preferable example of the alkoxycarbonyl group includes methoxycarbonyl group. When permeability through membrane needs to be increased, it is particularly preferable that acetoxymethyl group is used as the protective group of the carboxyl group so that the carboxyl group having the protective group is acetoxymethyloxycarbonyl group. The position of the carboxyl group which substitutes on the aryl ring or the heteroaryl ring is not particularly limited, and para-position is preferable when phenyl group is used as an aryl group.

In the group represented by the formula (A), it is preferred that m is 0 or 1 and n is 0. In this embodiment, both of X¹ and X² are preferably 2-pyridylmethyl groups, and further, when m is 1, X³ is preferred to be hydrogen atom. Y represents a single bond or -CO-, and Y is preferred to be a single bond. When Y represents a single bond, R³ is preferred to be a carboxy-substituted aryl group or a carboxy-substituted heteroaryl group, more specifically, R³ is preferred to be a carboxy-substituted phenyl group or a carboxy-substituted oxazolyl group.

The compounds of the present invention represented by the general formula (I) can exist as acid addition salts or base addition salts. Examples of the acid addition salts include: mineral acid salts such as hydrochloride, sulfate, and nitrate; and organic acid salts such as methanesulfonate, p-toluenesulfonate, oxalate, citrate, and tartrate. Examples of the base addition salts include: metal salts such as sodium salts, potassium salts, calcium salts, and magnesium salts; ammonium salts; and organic amine salts such as triethylamine salts. In addition, salts of amino acids such as glycine may be formed. The compounds or salts thereof according to the present invention may exist as hydrates or solvates, and these substances fall within the scope of the present invention.

The compounds of the present invention represented by the aforementioned formula (I) may have one or more asymmetric carbons depending on the types of the substituents. Stereoisomers such as optically active substances based on one or more asymmetric carbons and diastereoisomers based on two or more asymmetric carbons, as well as any mixtures of the stereoisomers, racemates and the like fall within the scope of the present invention.

Methods for preparing typical compounds of the present invention are shown in the following schemes. The preparation methods shown in the schemes are more specifically detailed in the examples of the specification. Accordingly, one of ordinary skill in the art can prepare any of the compounds of the present invention represented by the aforementioned general formula (I) by suitably choosing starting reaction materials, reaction conditions, reagents and the like based on these explanations, and optionally modifying and altering these methods.

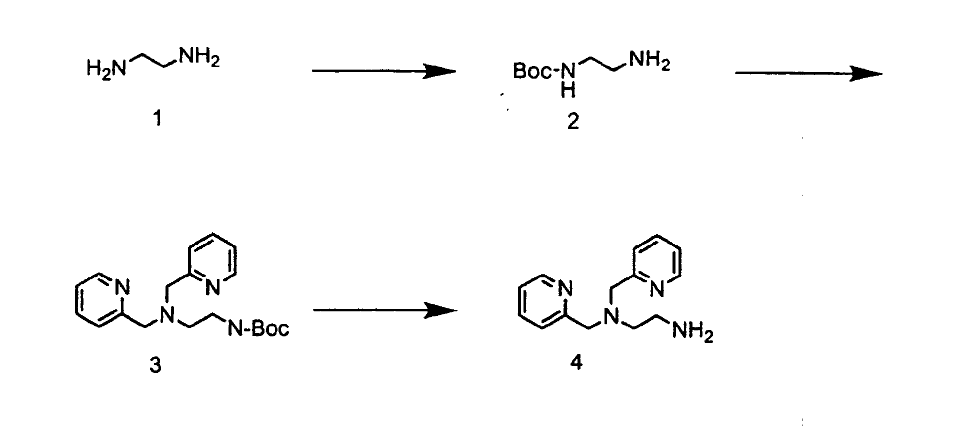

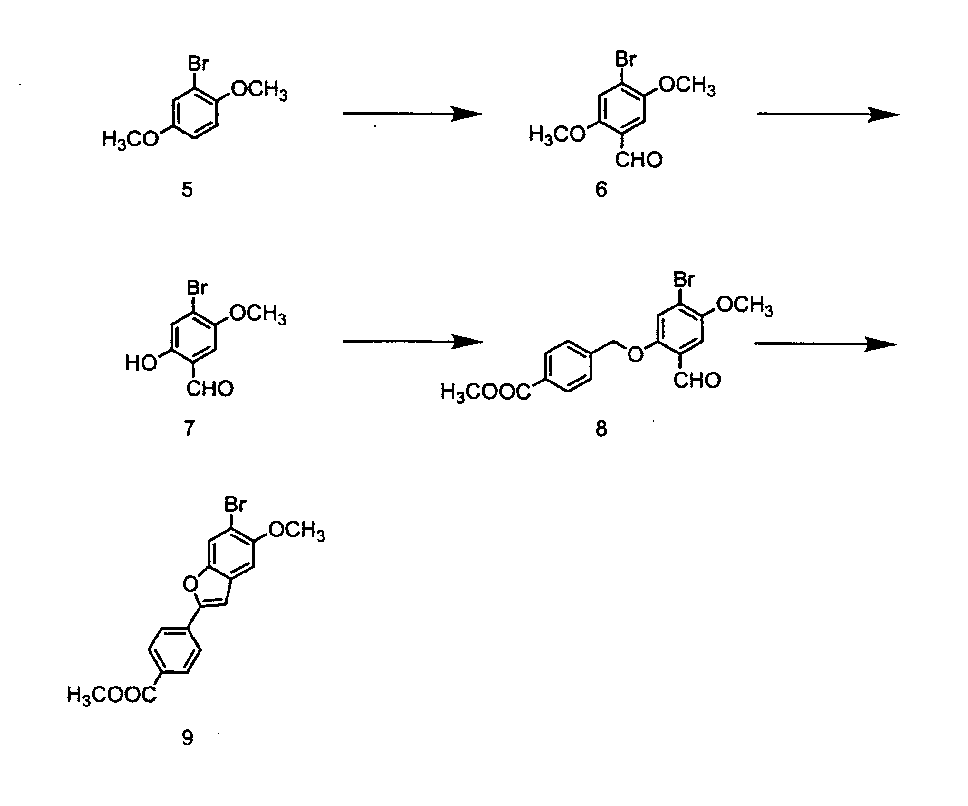

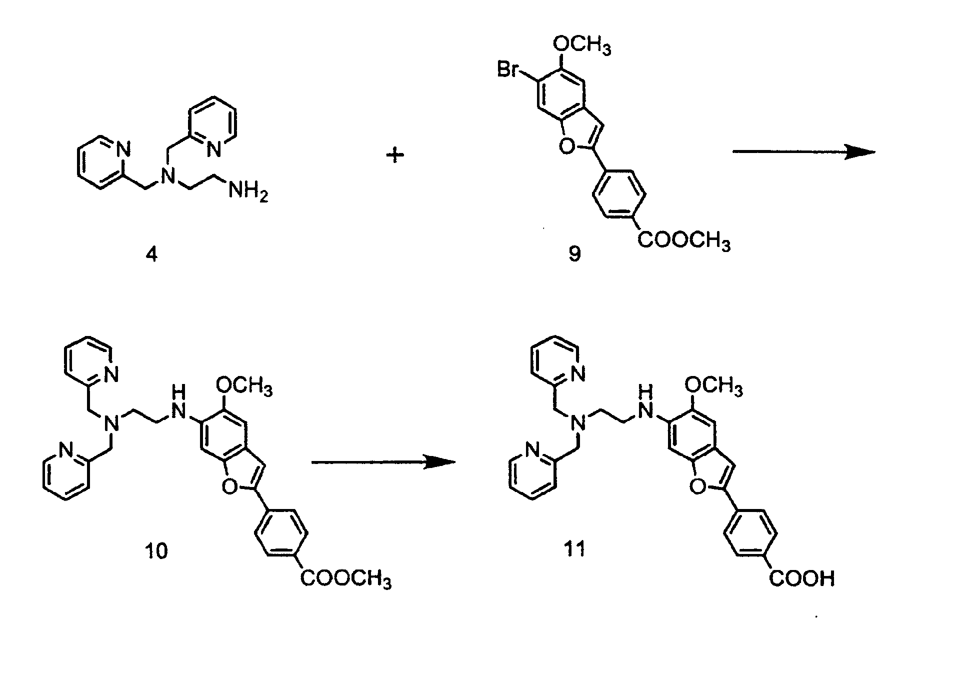

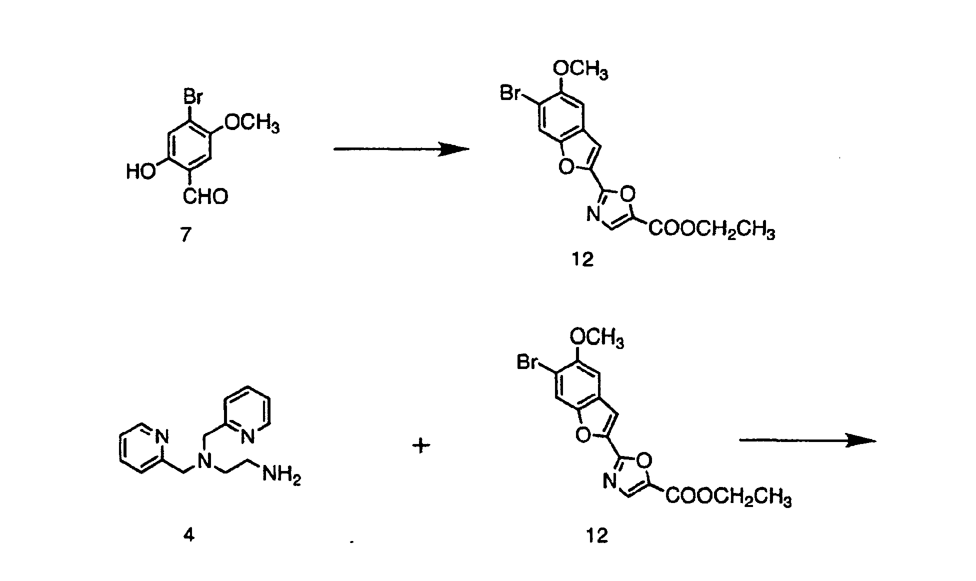

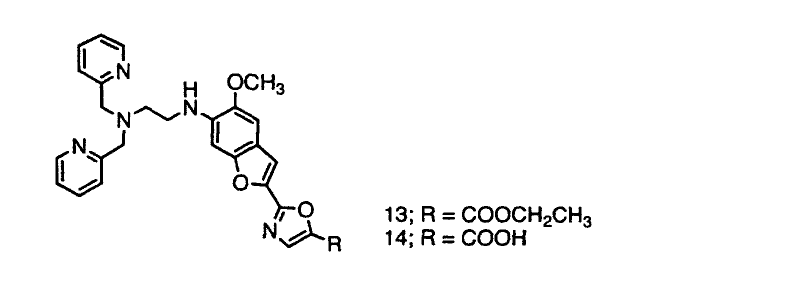

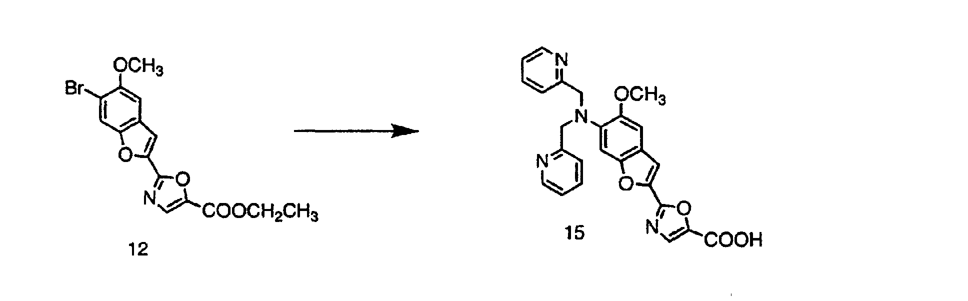

The compounds of the present invention represented by the aforementioned formula (I) or salts thereof are useful as fluorescent probes for zinc. The compounds of the present invention represented by the aforementioned formula (I) or salts thereof will give a remarkable wavelength shift of a peak in an excitation spectrum, when they forms zinc complexes by trapping zinc ions. The wavelength shift can be observed in general as much as about 20 nm width depending on the concentration of zinc ions. The shift can also be observed as a wavelength shift specific to a zinc ion without influence of other metal ions (for example, sodium ions, calcium ions, potassium ions, or magnesium ions). Therefore, by using the compound of the present invention as a fluorescent probe for zinc; and by selecting two appropriately different wavelengths to carry out excitation and measuring a ratio of the fluorescence intensities at the excitations, zinc ions can be measured by the ratio method. The two different wavelengths may be selected in such a manner that fluorescence intensity increases with increase of zinc ion concentration at one wavelength, whilst fluorescence intensity decreases with the increase of zinc ion concentration at the other wavelength. The details of the ratio method are described in the book by Mason W. T. (Mason W.T. in Fluorescent and Luminescent Probes for Biological Activity, Second Edition, Edited by Mason W.T., Academic Press). Specific examples of the measurement method using the compounds of the present invention are also shown in Examples of the specification.

The compounds of the present invention represented by the aforementioned general formula (I) or salts thereof are featured that they can specifically trap zinc ions and form complexes very rapidly. Accordingly, the compounds of the present invention represented by the aforementioned formula (I) or salts thereof are very useful as fluorescent probes for zinc for measurement of zinc ions in living cells or living tissues under a physiological condition. The term "measurement" used in the specification should be construed in its broadest sense, including quantitative and qualitative measurements.

A method for use of the fluorescent probe for zinc according to the present invention is not particularly limited, and the probe can be used in a similar manner to that of conventional zinc probes. In general, a substance selected from the group consisting of the compounds represented by the aforementioned general formula (I) and salts thereof is dissolved in an aqueous medium such as physiological saline or a buffered solution, or in a mixture of the aqueous medium and a water-miscible organic solvent such as ethanol, acetone, ethylene glycol, dimethylsulfoxide, and dimethylformamide, and then the resultant solution is added to a suitable buffered solution containing cells or tissues. The solution is excited at suitably selected two wavelengths, and each of fluorescence intensities may be measured.

For example, Compound 11 shown in the above scheme has the excitation wavelength at 354 nm, and the fluorescence wavelength at 532 nm. When the compound is used at 20 µM as a fluorescent probe for zinc, zinc ions at a concentration of about 20 µM or below are trapped, and as a result, a peak of an excitation spectrum will give about 20 nm blue shift depending on the concentration of zinc ions. Therefore, when this compound is used as a probe, excitation wavelengths such as 335 nm and 354 nm may be used, and the fluorescence intensity at each excitation wavelength is measured to calculate the ratio. The fluorescent probe for zinc according to the present invention may be combined with a suitable additive to use in the form of a composition. For example, the fluorescent probe for zinc can be combined with additives such as a buffering agent, a solubilizing agent, and a pH modifier.

### Examples

The present invention will be more specifically explained with reference to the following examples. However, the scope of the present invention is not limited to these examples. The compound numbers in the examples correspond to those used in the above schemes.

### Example 1

Compound (2) was prepared according to the method described in Journal of Organometalic Chemistry, 2000, 611, pp.586-592. Compound (2) (4.7 g: 29 mmol) was dissolved in 150 ml of ethanol, added with 12 g (0.12 mol) of sodium carbonate and 9.6 g (58 mmol) of 2-(chloromethyl) pyridine hydrochloride, and heated under reflux for one day. Ethanol was evaporated under reduced pressure, and the residue was suspended in a 2N aqueous sodium hydroxide solution, which was then extracted with dichloromethane. The organic layer was washed with saturated brine, and dried over potassium carbonate. The dichloromethane was evaporated under reduced pressure. The residue was purified by alumina column to obtain 9.9 g of Compound (3) (yield: quantitative).
Brown oil
¹H-NMR (CDCl₃, 300 MHz): 8.55 (m, 2H), 7.64 (m, 2H), 7.42 (d, 2H, J = 9.0), 7.16 (m, 2H), 5.80 (br, 1H), 3.87 (s, 4H), 3.23 (t, 2H, J = 6.0), 2.71 (t, 2H, J = 6.0)

Compound (3) (1.1 g) was dissolved in 25 ml of dichloromethane, added dropwise with 25 ml of trifluoroacetic acid on ice cooling. The mixture was stirred for one hour at room temperature, and the trifluoroacetic acid was evaporated under reduced pressure at 45 °C. The residue was added with 25 ml of 2N aqueous sodium hydroxide solution and extracted with dichloromethane. The organic layer was washed with saturated brine, and dried over potassium carbonate. The dichloromethane was evaporated under reduced pressure. The residue was purified by alumina column to obtain 0.64 g of Compound (4) (yield: 85%).
Brown oil
¹H-NMR (CDCl₃ , 300 MHz): 8.54 (m, 2H), 7.65 (m, 2H), 7.50 (d, 2H, J = 7.8), 7.15 (m, 2H), 3.85 (s, 4H), 2.80 (t, 2H, J = 6.0), 2.66 (t, 2H, J = 6.0), 1.42 (br, 2H)

2,5-Dimethoxybromobenzene (5) (7.0 ml :47 mmol) was dissolved in 160 ml of dichloromethane. The solution was added with 13 ml (0.12 mol) of titanium chloride (IV) under argon atmosphere at -78°C, subsequently with 8.2 ml (0.14 mol ) of dichloromethylmethy ether, and stirred at -78°C for one hour. The reaction mixture was gradually added to 600 ml of ice water and extracted with dichloromethane. The dichloromethane layer was washed with an aqueous saturated sodium hydrogencarbonate solution, water, and saturated brine, and then dried over sodium sulfate. The dichloromethane was evaporated under reduced pressure, and the residue was purified by silica gel column to obtain 9.9 g of Compound (6) (yield 87 %).
¹H-NMR (CDCl₃, 300 MHz): 10.40 (s, 1H), 7.34 (s, 1H), 7.25 (s, 1H), 3.91 (s, 3H), 3.90 (s, 3H)

Compound (6) (5.0 g) was dissolved in 130 ml of nitromethane. The solution was added with 80 ml of nitromethane saturated with zinc oxide, and further with 60 ml of dichloromethane solution of 1.0 M boron trichloride, and stirred at room temperature for 4 hours. The solution was further added with dichloromethane solution of 1.0 M boron trichloride (20 ml) and stirred at room temperature for 2 hours. The reaction mixture was added with 100 ml of water: ethanol= 1:1, stirred at room temperature for 30 minutes, and extracted with dichloromethane. The dichloromethane layer was washed with saturated brine, and then dried over sodium sulfate. The dichloromethane was evaporated under reduced pressure. The residue was purified by silica gel column to obtain 3.5 g of Compound (7) (yield 74 %).
¹H-NMR (CDCl₃, 300 MHz): 10.72 (s, 1H), 9.85 (s, 1H), 7.28 (s, 1H), 6.98 (s, 1H), 3.91 (s, 3H)

Compound (7) (2.0 g: 8.7 mmol) and 4-bromomethyl benzoic acid methyl ester (2.8 g: 12 mmol) were dissolved in 100 ml of dimethylformamide. The solution was added with potassium carbonate (4.8 g: 35 mmol) and stirred at 100 °C for two hours. After cooling down to room temperature, the solution was dissolved in 300 ml of ethyl acetate. The mixed solution was washed with water and an saturated brine, and then dried over sodium sulfate. The ethyl acetate was evaporated under reduced pressure. The residue was purified by silica gel column to obtain 1.9 g of Compound (8) (yield 58 %).
¹H-NMR (CDCl₃, 300 MHz): 10.47 (s, 1H), 8.09 (d, 2H, J = 8.0), 7.50 (d, 2H, J = 8.0), 7.37 (s, 1H), 7.30 (s, 1H), 5.20 (s, 2H), 3.94 (s, 3H), 3.91 (s, 3H)

Compound (8) (1.9 g: 5.0 mmol) was dissolved in 75 ml of dimethylformamide. The solution was added with 2.9 g of KF-Alumina (prepared by the method described in Bull. Chem. Soc. Jpn., 1983, 56, 1885-1886) , and stirred at 100°C for 3 hours. After filtration, the reaction mixture was added with ethyl acetate, and washed with water and saturated brine. After the solution was dried over sodium sulfate, ethyl acetate was evaporated under reduced pressure. The residue was purified by silica gel column to obtain 0.66 g of Compound (9) (yield 36 %).
¹H-NMR (CDCl₃, 300 MHz): 8.11 (d, 2H, J = 8.2), 7.89 (d, 2H, J = 8.2), 7.75 (s, 1H), 7.08 (s, 1H), 7.08 (s, 1H), 3.95 (s, 3H), 3.95 (s, 3H)

Compound (4) (0.33 g: 1.3 mmol) was dissolved in 20 ml of 1,4-dioxane. The solution was added with Compound (9) (0.16 g: 0.44 mmol), sodium-t-butoxide (89 mg: 0.92 mmol), palladium (II) [1,1'-bis(diphenylphosphino)ferrocene] dichloride (15 mg: 0.020 mmol), and 1,1'-bis(diphenylphosphino)ferrocene (23 mg: 0.042 mmol), and stirred at 100 °C for one hour. After addition of small amount of water, the reaction mixture was added with aqueous sodium hydrogencarbonate and dichloromethane, and the undissolved solids were filtered off by a glass filter. The filtrate was extracted with dichloromethane, and the extract was dried over sodium sulfate. The dichloromethane was evaporated under reduced pressure. The residue was purified by silica gel column to obtain 0.39 g of Compound (10) (yield 32 %).
Yellow solid.
¹H-NMR (CDCl₃, 300 MHz): 8.54 (m, 2H), 8.05 (d, 2H, J = 8.6), 7.80 (d, 2H, J = 8.6), 7.64 (m, 2H), 7.53 (d, 2H, J = 7.3), 7.14 (m, 2H), 7.01 (s, 1H), 6.90 (s, 1H), 6.64 (s, 1H), 3.96 (s, 3H), 3.96 (s,3H), 3.93 (s, 4H), 3.28 (t, 2H, J = 5.5), 2.96 (t, 2H, J = 5.5)
MS (FAB): 523 (M⁺+1)

In 25 ml of methanol, potassium hydroxide (1.0 g) was dissolved, and then 63 mg (0.12 mmol) of Compound (10) was dissolved. The solution was then stirred at 40 °C for 12 hours. The methanol was evaporated under reduced pressure, and then the residue was dissolved in 100 ml of water. The solution was added with hydrochloric acid so as to be acidic, and then the solution was added with an aqueous sodium hydroxide solution to adjust a pH to 7.0. The deposited solids were collected by filtration to obtain 43 mg of Compound (11) (yield 70 %). The obtained Compound (11) was recrystallized from ethyl acetate/ n-hexane.
Yellow solid.
¹H-NMR (CDCl₃, 300 MHz): 8.61 (m, 2H), 7.94 (d, 2H, J = 9.0), 7.65 (d, 2H, J = 9.0), 7.65 (m, 2H), 7.54 (d, 2H, J = 7.3), 7.20 (m, 2H), 6.89 (s, 1H), 6.81 (s, 1H), 6.59 (s, 1H), 3.96 (s, 3H), 3.94 (s, 4H), 3.33 (t, 2H, J = 5.5), 2.98 (t, 2H, J = 5.5), 1.57 (br, 1H)

Compound (7) (0.56 g: 2.4 mmol) was dissolved in 10 ml of dimethylformamide. The solution was added with ethyl 2-chloromethyloxazole-5-carboxylate (prepared by the method described in J. Biol. Chem., 1985, 260, 3440-3450) (0.46 g: 2.4 mmol) and potassium carbonate (1.3 g: 9.7 mmol), and stirred at 100°C for 1.5 hours. After neutralization with 2N hydrochloric acid, the reaction mixture was extracted with ethyl acetate, and the organic layer was washed with water and saturated brine. After the reaction mixture was dried over sodium sulfate, ethyl acetate was evaporated under reduced pressure. The residue was purified by silica gel column to obtain 0.51 g (1.4 mmol) of Compound (12). Yield 57 %.
¹H-NMR (CDCl₃, 300 MHz): 7.89 (s, 1H), 7.83 (s, 1H), 7.49 (s, 1H), 7.12 (s, 1H), 4.44 (q, 2H, J = 7.1 Hz), 3.96 (s, 3H), 1.42 (t, 3H, J = 7.1 Hz)
MS (EI): 364, 366

Compound (4) (0.20 g: 0.83 mmol) was dissolved in 10 ml of 1,4-dioxane. The solution was added with Compound (12) (0.10 g: 0.27 mmol), sodium-t-butoxide (54 mg: 0.56 mmol), palladium (II) [1,1'-bis(diphenylphosphino)ferrocene] dichloride (5 mol%), and 1,1'-bis(diphenylphosphino)ferrocene (10 mol%), and stirred under argon atmosphere at 80°C for two hours. The reaction mixture was evaporated under reduced pressure. The residue was purified by NH silica gel column to obtain 5.0 mg (9.5 mmol) of Compound (13). Yellow oil. Yield 3.5 %.
¹H-NMR (CDCl₃, 300 MHz): 8.53 (d, 2H, J = 5.0 Hz), 7.84 (s, 1H), 7.63 (m, 2H), 7.51(d, 2H, J = 7.9 Hz), 7.43 (s, 1H), 7.14 (m, 2H), 6.90 (s, 1H), 6.59 (s, 1H), 5.39 (brs, 1H), 4.41 (q, 2H, J = 7.1 Hz), 3.97 (s, 3H), 3.91 (s, 4H), 3.24 (t, 2H, J = 5.9 Hz), 2.95 (t, 2H, J = 5.9 Hz), 1.41 (t, 3H, J = 7.1 Hz)
¹³C-NMR (75 MHz, CDCl₃): 159.23, 157.86, 157.46, 153.11, 149.10, 145.82, 141.48, 140.17, 140.06, 136.40, 135.63, 123.03, 122.12, 115.68, 111.29, 100.55, 91.86, 61.47, 60.40, 56.00, 52.31, 41.04, 14.31
HRMS (FAB⁺) Calcd for (M+H⁺) m/z 528.2247, Found 528.2255

Compound (4) (0.20 g: 0.83 mmol) was dissolved in 10 ml of 1,4-dioxane. The solution was added with Compound (12) (0.10 g: 0.27 mmol), sodium-t-butoxide (54 mg: 0.56 mmol), palladium (II) (1,1'-bis(diphenylphosphino)ferrocene] dichloride (5 mol%), and 1,1'-bis(diphenylphosphino)ferrocene (10 mol%), and stirred under argon atmosphere at 80°C for two hours. The reaction mixture was added with several ml of methanol and stirred at room temperature for 15 minutes. The reaction mixture was neutralized with 2N hydrochloric acid, and the solvent was evaporated under reduced pressure. The residue was purified by reversed phase HPLC to obtain 20 mg (40 nmol) of Compound (14). Yellow oil. Yield 15 %. The compound was precipitated as hydrochloride, and used for measurements.
¹H-NMR (CD₃OD, 300 MHz): 8.79 (d, 2H, J = 5.9 Hz), 8.48 (m, 2H), 8.10 (d, 2H, J = 7.8 Hz), 7.92 (s, 1H), 7.90 (m, 2H), 7.56 (s, 1H), 7.19 (s, 1H), 6.89 (s, 1H), 4.41 (s, 4H), 3.95 (s, 3H), 3.52 (t, 2H), 3.05 (t, 2H)
¹³C-NMR (75 MHz, CD₃OD): 160.27, 158.60, 154.03, 153.50, 148.54, 147.49, 143.68, 141.32, 141.15, 140.30, 135.87, 125.72, 125.61, 117.79, 112.59, 102.36, 92.55, 58.99, 56.71, 53.98, 40.18
HRMS (FAB⁺) Calcd for (M+H⁺) m/z 500.1934, Found 500.1936

Di-(2-picolyl)amine (0.20 g: 0.96 mmol) was dissolved in 10 ml of 1,4-dioxane. The solution was added with Compound (12) (0.10 g: 0.27 mmol), sodium-t-butoxide (54 mg: 0.56 mmol), palladium (II) [1,1'-bis(diphenylphosphino)ferrocene] dichloride (5 mol%), and 1,1'-bis(diphenylphosphino)ferrocene (10 mol%), and stirred under argon atmosphere at 100 °C for 3.5 hours. The reaction mixture was added with several ml of methanol and stirred at room temperature for 15 minutes. The reaction mixture was neutralized with 2N hydrochloric acid, and the solvent was evaporated under reduced pressure. The residue was purified by reversed phase HPLC to obtain 2 mg (4.4 nmol) of Compound (14). Yellow oil. Yield 1.6 %. The compound was precipitated as hydrochloride, and used for measurements.
¹H-NMR (CD₃OD, 300 MHz): 8.76 (d, 2H, J = 5.1 Hz), 8.39 (m, 2H), 7.99 (d, 2H, J = 8.4 Hz), 7.83 (s, 1H), 7.82 (m, 2H), 7.46 (s, 1H), 7.39 (s, 1H), 7.22 (s, 1H), 4.88 (s, 4H), 3.72 (s, 3H)

### Example 2: Fluorescence characteristics of Compound (11)

Fluorescence characteristics of Compound (11) were studied. Compound (11) was dissolved in 100 mM HEPES buffer (pH 7.4, containing 0.4% DMSO as co-solvent) up to 20 µM and measured excitation spectra.

### Measurement conditions:

Hitachi F-4500 fluorescence measurement apparatus
Slit: Ex, Em 2.5 nm
Scanning rate: 240 nm/min
Photomultiplier voltage: 950 V
Measurement temperature: 25°C
Fluorescence characteristics of Compound (11) before and after addition of zinc ions (20 µM ZnSO₄) is shown in the following Table 1.

**Table 1**

| | Ex(nm) | Em(nm) |
|---|---|---|
| Before addition of Zn²⁺ | 354 | 532 |
| After addition of Zn²⁺ | 335 | 528 |

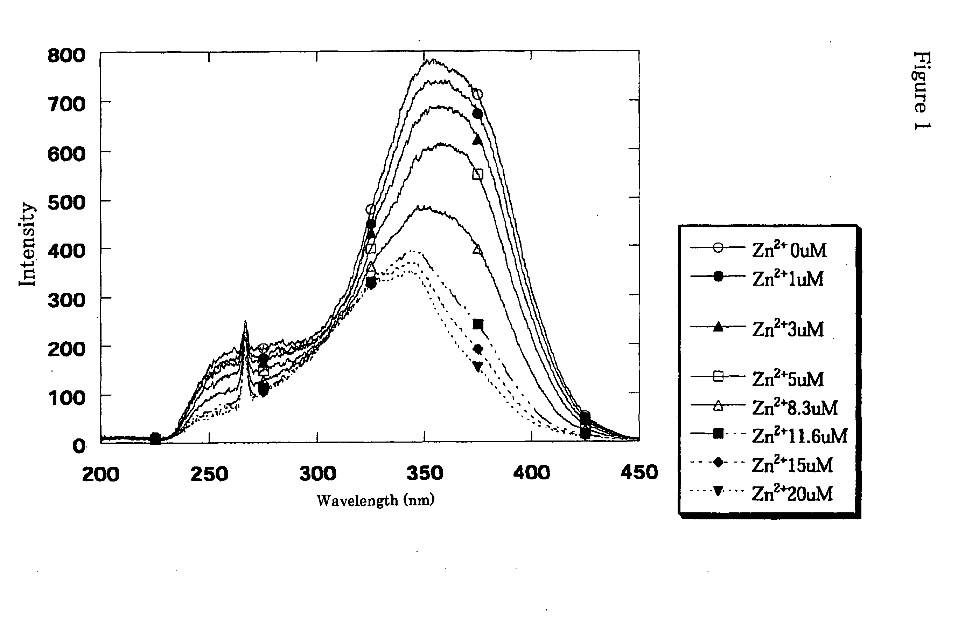

A wavelength shift of about 20 nm was observed for Compound (11) because of a coordination of the compound to a zinc ion. In the presence of 20 µM of Compound (11), changes of excitation spectra over the change of the concentration of zinc ions are shown in Figure 1. A shift of maximum wavelength of the excitation spectra to a shorter wavelength was observed depending on concentrations of zinc ions.

### Example 3: Ratio measurement of Compound (11)

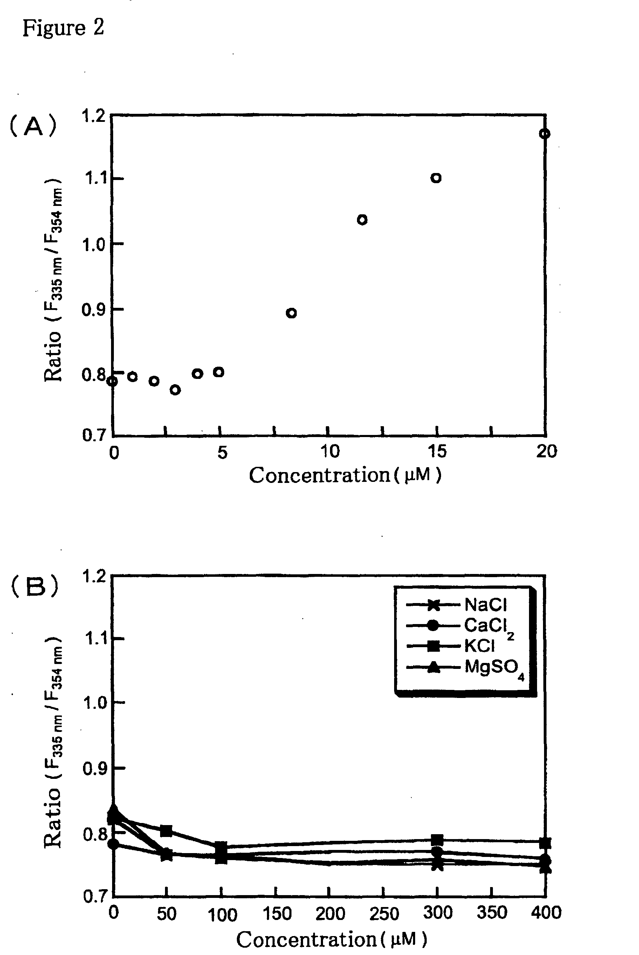

To a 20 µM solution of Compound (11) in 100 mM HEPES buffer (pH 7.4), ZnSO₄ was added up to 20 µM. Change of the ratio of fluorescence intensities at excitation wavelengths at 335 nm and 354 nm with the fluorescence wavelengths fixed at 530 nm are shown (Figure 2 (A)). To a 20 µM solution of Compound (11) in 100 mM HEPES buffer (pH 7.4), sodium ions, calcium ions, potassium ions, or magnesium ions were added up to 400 µM. Changes of the ratio of fluorescence intensities at excitation wavelengths at 335 nm and 354 nm with the fluorescence wavelengths fixed at 530 nm are shown (Figure 2 (B)). The ratio changed concentration dependent manner of zinc ions when zinc ions were added, whereas no change of the ratio was observed when the other ions were added. These results indicate that Compound (11) has high selectivity to zinc ions.

### Example 4: Fluorescence characteristics and ratio measurement of Compound (14) and (15)

Studies of fluorescence characteristics and the ratio measurements of Compound (14) and (15) were conducted in the same manners as those described in Example 2 and Example 3. Fluorescent characteristics of Compound (14) and (15) before and after addition of zinc ions (20 µM ZnSO₄) are shown in the following Table 2.

**Table 2**

| | Compound(14) | | Compound(15) | |
|---|---|---|---|---|
| | Ex(nm) | Em(nm) | Ex(nm) | Em(nm) |
| Before addition of Zn²⁺ | 365 | 495 | 355 | 495 |
| After addition of Zn²⁺ | 335 | 495 | 325 | 445 |

Similar to the result of Compound (11), a wavelength shift of about 30 nm was observed for each of Compound (14) and (15) because of a coordination of each of the compounds to a zinc ion. Addition of sodium ions, calcium ions, potassium ions, or magnesium ions caused no change in the ratio, which verifies that the compounds indicate high selectivity to a zinc ion.

### Example 5: Spectrum measurement of Compound (14) and (15)

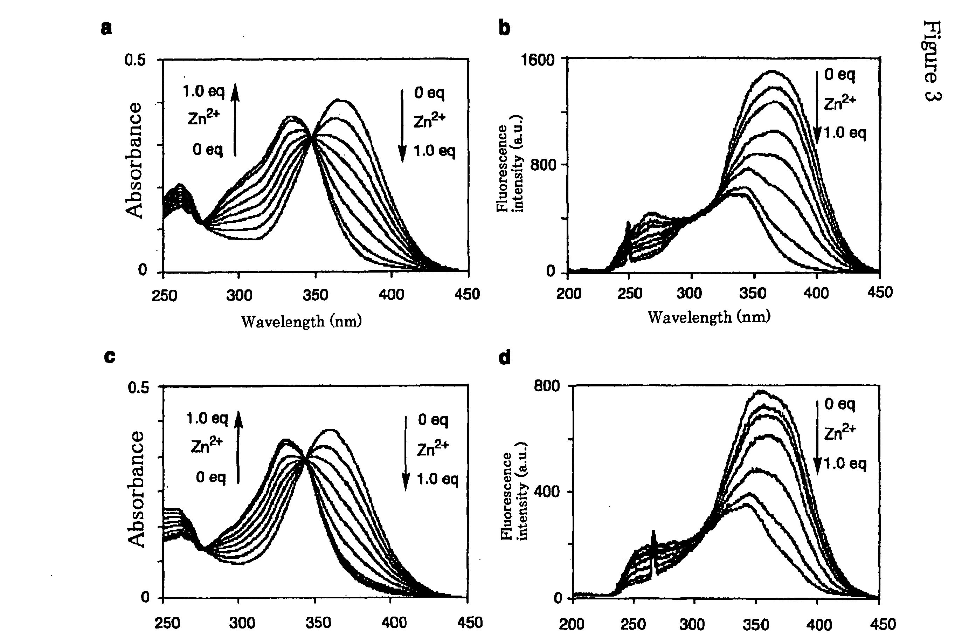

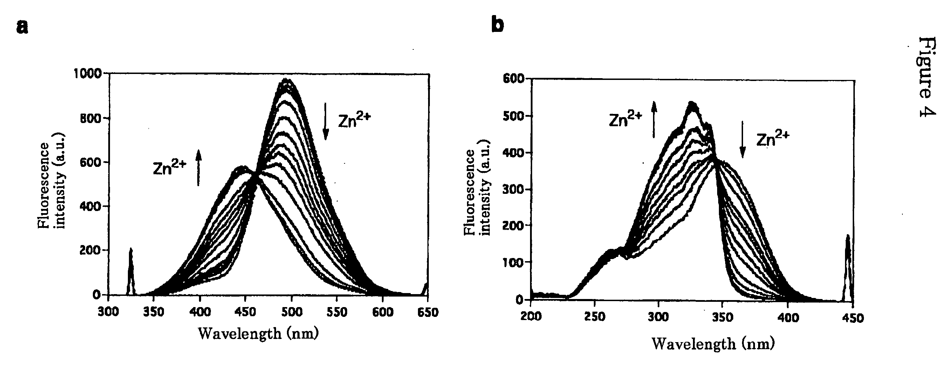

To a 15 µM solution of Compound (14) in 100 mM HEPES buffer (pH 7.4), ZnSO₄ was added up to 15 µM. Spectra of the solution were measured under the measurement condition described in Example 2. The same spectral measurement was also conducted for Compound (11) as a reference. In Figure 3, a) UV spectrum change of Compound (14), b) excitation spectra of Compound (14) with fluorescence wavelengths fixed at 495 nm, c) UV spectrum change of Compound (11), and d) excitation spectra of Compound (11) with fluorescence wavelengths fixed at 530 nm are shown. Further, to a 10 µM solution of Compound (15) in 100 mM HEPES buffer (pH 7.4), ZnSO₄ was added up to 200 µM. Spectra were measured under the condition described in Example 2. Figure 4 shows a) Fluorescence spectra of Compound (15) with excitation wavelengths fixed at 325 nm, and b) excitation spectra of Compound (15) with fluorescence wavelengths fixed at 445 nm.

The addition of zinc ions caused decreases of the absorbance at 365 nm for Compound (14) and that at 354 nm for Compound (11), and caused increases of the absorbance at 335 nm for both of Compound (14) and (11) (Figure 3 a) and c)). At the same time, the addition of zinc ions caused decreases of the fluorescence excited at 365 nm for Compound (14) and that excited at 354 nm for Compound (11) (Figure 3 b) and d)). Further, as for Compound (15), the addition of zinc ions caused a decrease of fluorescence at 495 nm and an increase of fluorescence at 445 nm (Figure 4a)), whereas the addition of zinc ions caused a decrease of fluorescence intensity excited at 355 nm and an increase of fluorescence intensity on excited at 325 nm (Figure 4b)). Therefore, it is shown that Compound (14) and (15), as well as Compound (11), can be used for the measurement of zinc ions by the ratio method.

### Example 6: Zinc ion measurement in living cells by using Compound (13)

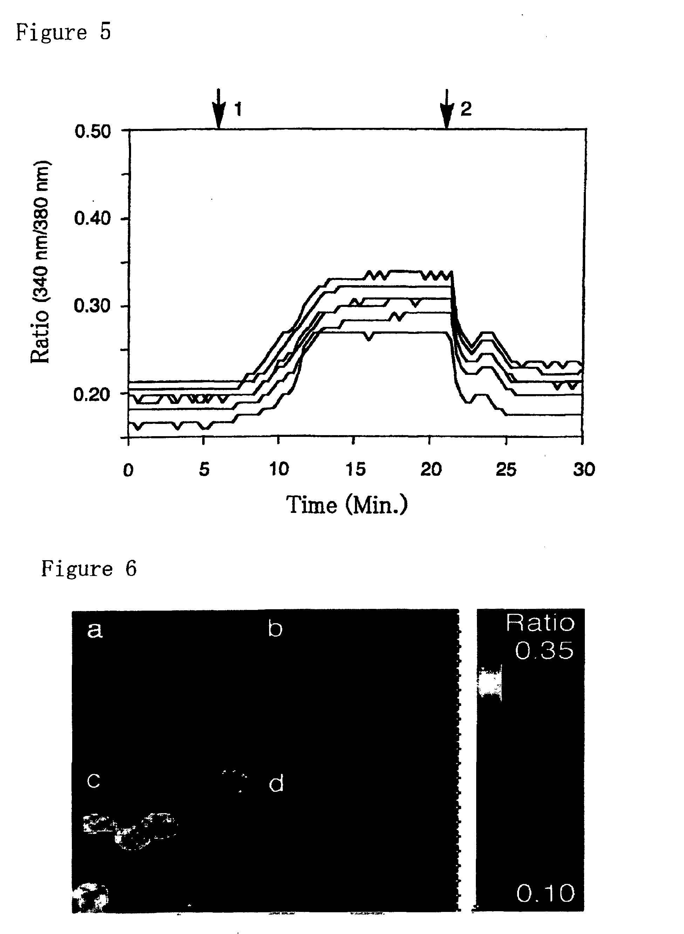

Changes in zinc ion concentration in living cells were measured by imaging. Macrophage (RAW264.7) was incubated in PBS buffer containing 10 µM of Compound (13) at room temperature for 30 minutes. The extracellular solution was changed to PBS buffer free from Compound (13), and then the ratio change of fluorescence intensities excited at 340 nm and 380 nm was observed under fluorescence microscope. As shown in Figure 5, pyrithione (ionophore selective to zinc ion) and ZnSO₄ were added to the extracellular solution so as to be10 µM and 150 µM, respectively, at the time point of 5 minutes after the start of the measurement (Figure 5, Arrow 1). After additional 15 minutes, TPEN (membrane permeable zinc ion chelating agent) was added to the extracellular solution so as to be 400 µM (Figure 5, Arrow 2). Figure 6 shows image of transmitted light through cells and changes in fluorescence intensity ratio which are pictured using pseudo-color technique.

An increase in the ratio after the addition of pyrithione and ZnSO₄, and a decrease in the ratio after the addition of TPEN were observed for all of six macrophages within the visual filed

### Industrial Applicability

The compound of the present invention is useful as a fluorescent probe for measurement of zinc. The compound of the present invention will give a wavelength shift of a peak in an excitation spectrum after formation of a complex with a zinc ion, and therefore, the zinc ions can be precisely measured by the ratio method by using different two excitation wavelengths. In a measurement of zinc ion concentration by the ratio method, influences of a concentration of fluorescent probe, per se, and intensities of excitation light are negligible, and measurement errors due to localization, concentration change, and discoloration of fluorescent probe per se can be eliminated. Accordingly, the compound of the present invention is extremely useful as a probe for a precise measurement of zinc ions in living organisms.

## Claims

1. A compound represented by the following general formula (I) or a salt thereof: 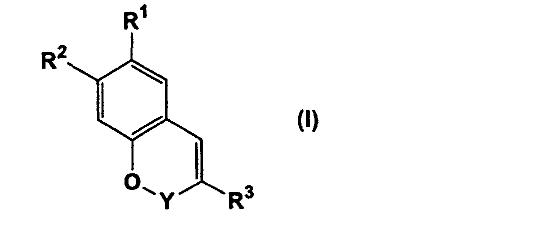 wherein R¹ represents hydrogen atom, an alkyl group, an alkoxy group, or hydroxy group; R² represents a group represented by the following formula (A): 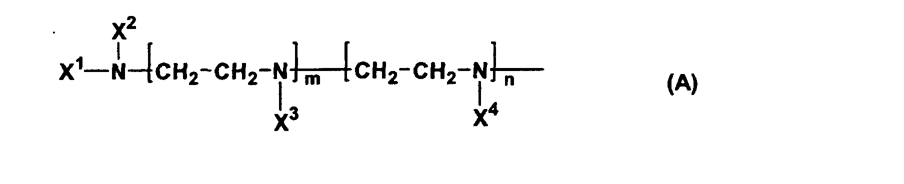 wherein X¹, X², X³, and X⁴ each independently represents hydrogen atom, an alkyl group, or 2-pyridylmethyl group, and m and n each independently represents 0 or 1; Y represents a single bond or -CO-; R³ represents a carboxy-substituted aryl group, a carboxy-substituted heteroaryl group, benzothiazol-2-yl group, or 5-oxo-2-thioxo-4-imidazolyzinylidenmethyl group.

2. The compound or a salt thereof according to claim 1, wherein m is 0 or 1 and n is 0.

3. The compound or a salt thereof according to claim 2, wherein both of X¹ and X² are 2-pyridylmethyl groups, and when m is 1, X³ is hydrogen atom.

4. The compound or a salt thereof according to any one of claims 1 to 3, wherein Y is a single bond.

5. The compound or a salt thereof according to any one of claims 1 to 4, wherein R¹ is methoxy group.

6. The compound or a salt thereof according to any one of claims 1 to 5, wherein R³ is a carboxy-substituted aryl group or a carboxy-substituted heteroaryl group.

7. The compound or a salt thereof according to claim 6, wherein the carboxyl group substituting on the aryl ring or the heteroaryl ring has a protective group.

8. The compound or a salt thereof according to claim 7, wherein the carboxyl group having a protective group is methoxycarbonyl group or acetoxymethyloxycarbonyl group.

9. The compound or a salt thereof according to claim 1, wherein m is 1, n is 0, both of X¹ and X² are 2-pyridylmethyl groups, X³ is hydrogen atom, Y is a single bond, R¹ is methoxy group, and R³ is p-carboxyphenyl group.

10. The compound or a salt thereof according to claim 1, wherein m is 1, n is 0, both of X¹ and X² are 2-pyridylmethyl groups, X³ is hydrogen atom, Y is a single bond, R¹ is methoxy group, and R³ is 5-carboxyoxazol-2-yl group.

11. The compound or a salt thereof according to claim 1, wherein m is 0, n is 0, both of X¹ and X² are 2-pyridylmethyl groups, Y is a single bond, R¹ is methoxy group, and R³ is 5-carboxyoxazol-2-yl group.

12. The compound or a salt thereof according to claim 1, wherein m is 1, n is 0, both of X¹ and X² are 2-pyridylmethyl groups, X³ is hydrogen atom, Y is a single bond, R¹ is methoxy group, and R³ is p-acetoxymethyloxycarbonylphenyl group.

13. The compound or a salt thereof according to claim 1, wherein m is 1, n is 0, both of X¹ and X² are 2-pyridylmethyl groups, X³ is hydrogen atom, Y is a single bond, R¹ is methoxy group, and R³ is 5-acetoxymethyloxycarbonyloxazol-2-yl group.

14. The compound or a salt thereof according to claim 1, wherein m is 0, n is 0, both of X¹ and X² are 2-pyridylmethyl groups, Y is a single bond, R¹ is methoxy group, and R³ is 5-acetoxymethyloxycarbonyloxazol-2-yl group.

15. A fluorescent probe for zinc which comprises the compound or a salt thereof according to any one of claims 1 to 14.

16. A zinc complex which is formed with the compound or a salt thereof according to any one of claims 1 to 14 and a zinc ion.

17. An agent for measuring zinc ions which comprises the compound or a salt thereof according to any one of claims 1 to 14.

18. A method for measuring zinc ions which comprises the following steps of:
(a) reacting the compound or a salt thereof according to any one of claims 1 to 14 with a zinc ion; and
(b) measuring fluorescence intensity of a zinc complex produced in the above step (a).

19. The method according to claim 18, wherein the measurement is conducted by the ratio method.

20. A method for measuring zinc ions which comprises the following steps of:
(a) allowing the compound or a salt thereof according to claims 7 or 8 to be taken up into cells; and
(b) measuring fluorescence intensity of a zinc complex produced by a reaction, with zinc ions, of a compound or a salt thereof according to claim 6 (provided that the carboxyl group does not have a protective group) which is generated by hydrolysis of said compound or a salt thereof after being taken up into cells.

21. The method according to claim 20, wherein the measurement is conducted by imaging.
